(19) Europäisches Patentamt
European Patent Office
Office européen des brevets

(11) EP 3 471 107 A1

(12) EUROPEAN PATENT APPLICATION

(43) Date of publication:
17.04.2019 Bulletin 2019/16

(51) Int Cl.:
G16H 50/50 (2018.01)    G16H 50/80 (2018.01)

(21) Application number: 17196137.8

(22) Date of filing: 12.10.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Fresenius Medical Care Deutschland
GmbH
61352 Bad Homburg (DE)

(72) Inventors:
• TSCHULENA, Ulrich
  60439 Frankfurt/Main (DE)
• BELLOCCHIO, Francesco
  26852 Casaletto (LO (IT)
• NERI, Luca
  20144 Milano (MI) (IT)
• ARMSEN, Wencke
  65817 Eppstein (DE)
• BARBIERI, Carlo
  26013 Crema (CR) (IT)

(74) Representative: Lindemann, Robert
Fresenius Medical Care AG & Co. KGaA
Global Intellectual Property
Else-Kröner-Straße 1
61352 Bad Homburg (DE)

(54) **MEDICAL DEVICE AND COMPUTER-IMPLEMENTED METHOD OF PREDICTING RISK, OCCURRENCE OR PROGRESSION OF ADVERSE HEALTH CONDITIONS IN TEST SUBJECTS IN SUBPOPULATIONS ARBITRARILY SELECTED FROM A TOTAL POPULATION**

(57)    A computer-implemented method of generating a generalized model for adaptively predicting occurrence or progression of a first adverse health condition for a first subpopulation arbitrarily selected from a total population, includes extracting information about characterizing features of a plurality of second subpopulations, about occurrences and/or severity of the first adverse health condition found therein and/or about corresponding prognostic results, from a plurality of publications. One or more of the characterizing features are associated with corresponding first factors indicating a relation with an adverse or beneficial contribution of the characterizing feature to the occurrence or progression of the first adverse health condition, and with corresponding second factors indicating the relative frequency of occurrence in the respective second subpopulation considered in the respective publication. The characterizing features and their first and second factors are combined into a generalized model for the total population, including calculating a baseline risk of patient. The generalized model is stored on a computer accessible and readable medium in a retrievable manner.

Fig. 1

**Description**

**Technical Field**

The present method and apparatus relates to predicting occurrence or progression of adverse health conditions in test subjects.

**Background**

Medical-related information may originate from a plurality of different sources, such as clinical data or non-clinical data. Medical-related information may be used by health care professionals for the prescription and analysis of tests and/or for the diagnosis and treatment of diseases or medical events or, more generally, adverse health conditions. Medical-related information may also be used for assessing the risk of acquiring diseases, of existing diseases to aggravate, or of suffering from an adverse medical event. Health Risk Forecasting is a procedure aiming to analyze the probability of occurrence of a certain type of medical risk based on certain types of medical-related information. For example, Health Risk Forecasting can be used to analyze the probability of acquiring a lung disease based on whether or not a person is a smoker.

A number of studies and clinical tests have been published in which the progression of adverse health conditions and their ultimate outcome has been recorded along with various medical and other parameters characterizing the studied subjects. Some of the studies tried to find numerical algorithms for predicting the occurrence and progression of adverse health conditions and their ultimate outcome by aligning the medical and other parameters of a test subject with those that have been recorded earlier.

Some algorithms suffer from being based on data obtained from peer groups that are not representatively selected across the entire population. Thus, their predictive potential for test subjects whose medical and other parameters do not correspond to those of the peer group is limited. Additionally, certain current algorithms cannot promptly or easily be directly updated with emerging scientific evidence concerning certain medical-related information.

**Summary**

It is, therefore, desirable to provide a computer-implemented method of generating a generalized model for adaptively predicting occurrence or progression of a first adverse health condition for a first subpopulation arbitrarily selected from a total population. Further, it is desirable to provide a computer-implemented method of adaptively predicting occurrence or progression of a first adverse health condition for a first subpopulation arbitrarily selected from a total population. Yet further it is desirable to provide a medical device implementing the computer-implemented method of generating a generalized model and a medical device implementing the computer-implemented method of adaptively predicting occurrence or progression of a first adverse health condition for a first subpopulation arbitrarily selected from a total population.

The expression first adverse health condition is used herein to describe one or more from the non-exclusive list comprising cardiovascular event (CVE), chronic kidney disease (CKD), all cause hospitalizations, fractures, injuries, falls, infections, need for renal replacement therapy (e.g. dialysis or transplantation), end-stage renal disease, acute kidney injury, graft failure, vascular access complications, other disease outcome, etc.

The expression first subpopulation as used herein refers to an individual having traits, attributes, or other characterizing features which allow distinguishing the individual from other members of a total population. Instead of referring to an individual it may also refer to a group of individuals sharing the same traits, attributes, or other characterizing features, distinguishing the group from other members of the total population. The total population comprises a plurality of second subpopulations, e.g., subpopulations considered in respective scientific studies, in literature or the like. The total population may comprise all second subpopulations for which data is available for analysis. Different second subpopulations may or may not share one or more traits, attributes or other characterizing features with the first subpopulation or other second subpopulations. However, each subpopulation will differ in at least one trait from any other subpopulation. Second subpopulations separately analyzed in individual studies or pieces of literature in respect to the same adverse health conditions may be partially overlapping or may even be identical.

As stated above the expression characterizing features as used herein refers to traits, attributes, which allow distinguishing an individual or group of individuals from other individuals or groups of individuals. Characterizing features may include general information characterizing individuals or a population, genetic information, e.g. obtained from genetic marker analysis systems, medical events and states, treatments, diagnosis, and prognosis characterizations, etc. The characterizing features may further include demographic data, e.g., age, race, sex, work place, environmental factors, residence, life style, etc., self-reported data, e.g., from surveys captured intermittently from individuals or members of a population, prescription drug information, e.g., types and/or amount of prescription drugs taken by an individual or a population, data obtained from diagnostic records, e.g., clinical tests and results, and treatment data, e.g., illness, treatment, hospital, and/or doctor, etc. Medical data may be obtained from any kind of routine or advanced diagnostic test including but not limited to any imaging technique (e.g. magnetic resonance imaging, sonography, x-ray, CT scan, scintigraphy, etc), electrophysiology testing, physical examination results, immunoassays and radioimmunoassay systems, biochemistry, polymerase (PCR) chain reaction analysis systems, chromatography analysis systems, and/or receptor assay systems, etc. Data from other analysis systems, such as tissue analysis systems, cytology and tissue typing systems, and immunocytochemistry and histopathological analysis systems may also be included. The characterizing features may be time-invariant or relate to current and/or past time instants. The data may also be provided as a time series or as a derivative thereof, indicating a change over time.

In accordance with an aspect the method of generating a generalized model for adaptively predicting occurrence or progression of a first adverse health condition arbitrarily selected from a total population described herein includes extracting information about characterizing features of a plurality of second subpopulations, about occurrences and/or severity of the first adverse health condition found therein and/or about corresponding prognostic results, from a plurality of publications and/or primary clinical data recorded in electronic databases and elaborated according to probabilistic statistical models. One or more characterizing features, e.g. those known to be useful in the respective context, may be provided as further input for initializing the extraction, but this is not a strict requirement, since the extraction itself may identify other or further characterizing features as also or equally relevant, or even or more relevant.

The term publication is used herein to describe scientific studies and papers, general literature or literature with a focus on health issues, data sets correlating health issues with patient's properties that can be unambiguously linked with one or more patients: Such properties include but are not limited to age, gender, height, weight, BMI, use of substances or alcohol, smoking, history of hypertension or hypotension, diabetes, CKD stage, history of cerebrovascular disease, coronary artery disease, peripheral artery disease, chronic heart failure, chronic obstructive pulmonary disease, autoimmune disorders, anxiety/depression, cancer, liver disease, BMI, albumin, glucose, HDL, LDL, Triglycerides, CRP, IL-6, serum uric acid, HsTNT, Phosphate, iPTH, proteinuria and albuminuria, other known chronic diseases, past cured diseases, behavior or lifestyle, psychological profiles, morphological characteristics assessed via any imaging technique, or functional characteristics assessed via any appropriate diagnostic testing.

The information about characterizing features extracted from the plurality of publications and/or primary clinical data recorded in electronic databases and elaborated according to probabilistic statistical models may comprise those patient's properties which show a positive or negative correlation with regard to the first adverse health condition.

The method further includes associating, based on data from each of the plurality of publications and/or primary clinical data recorded in electronic databases and elaborated according to probabilistic statistical models, one or more of the characterizing features identified therein with corresponding first factors indicating a relation with an adverse or beneficial contribution of the characterizing feature to the occurrence or progression of the first adverse health condition. The first factors may represent, e.g., effect size measures such as odds ratios, hazard ratios, relative risks.

The method further includes associating, from each of the plurality of publications and/or primary clinical data recorded in electronic databases and elaborated according to probabilistic statistical models, one or more of the characterizing features identified therein with corresponding second factors indicating the relative frequency of occurrence, or prevalence, in the respective second subpopulation considered in the respective publication. The combination of data on characterizing features and associated first and second factors over a plurality of publications and/or primary clinical data recorded in electronic databases and elaborated according to probabilistic statistical models may provide an indication for a likelihood of occurrence or progression in the total population.

The relation indicated by the first factor may express a risk increase or decrease for occurrence or progression of the first adverse health condition in a patient, e.g. expressed with respect to a reference population, or as an absolute risk.

The method further includes combining the characterizing features and their first and second factors into a generalized model for the total population, wherein combining includes calculating a baseline risk of a patient and estimating the conditional probability of a first adverse health condition for all the potential configurations of known health states contributing to the first adverse health event risk. A baseline risk may be considered a general risk for a patient in which all identified and known risk factors are absent.

The combination of the characterizing features and their first and second factors may follow the steps exemplarily described hereafter.

In the case of effect sizes expressed as odds ratios, input to the process is

*adverse outcome incidence I*

*risk factor prevalence $P_1, P_2, ..., P_n$*

*effect size measures: $OR_1, OR_2, ..., OR_n$*

Output of the process is conditional probabilities of risk factors

$P_{RF\_1}, P_{RF\_2}, ... P_{RF\_n}$

for a given outcome

The procedure may be briefly summarized as

1. Get I
2. Get $P_1, P_2, ..., P_n$
3. Get $OR_1, OR_2, ..., OR_n$
4. Compute for each risk factor the contribution to the outcome risk as

$$W\_RF_i = ln(OR_i) \times P_i$$

5. Compute base risk as $\lambda = -ln((1-I)/I) - \Sigma_i W\_RF_i$
6. Compute the probability of the outcome given each risk factor as

$$P\_O_{RF\_i} = exp(\Sigma_{j \neq i} W\_RF_j + ln(OR_i) + \lambda)/(1 + exp(\Sigma_{j \neq i} W\_RF_j + ln(OR_i) + \lambda))$$

7. Compute the joint probability of outcome and each risk factor as

$$P\_O\_RF_i = P\_O_{RF\_i} \times P_i \text{(Bayes' theorem)}$$

8. Compute the probability of each risk factor given the outcome as

$$P_{RF\_i} = P\_O\_RF_i / I$$

The method further includes storing the generalized model in a retrievable manner on a computer accessible and readable medium.

In other words, the aspect of the method presented above selects the most relevant information from a vast array of available publications and/or primary clinical data recorded in electronic databases and elaborated according to probabilistic statistical models, like clinical and patient-reported data, assesses the contribution of patient-specific health-state attributes to future risk, creates a generalized model including a baseline reference, and stores the model for future application using a subpopulation's attributes as input values.

In an aspect of the method weights are derived and associated with the first and/or second factors. The weights may be derived from data from a single publication or with respect to a plurality of publications, or a mixture of a plurality of publications and primary clinical data recorded in multiple electronic databases and elaborated according to probabilistic statistical models.

In an aspect of the method extracting includes automatic extraction using one or more of electronic text processing, optical character recognition, natural language processing, but also manual data entry. In case of automatic extraction of characterizing features rule-based extraction or extraction assisted by artificial intelligence as well as other methods may be used.

In an aspect of the method the characterizing features and the associated factors are adjusted, ranked and/or selected for the quality of the publication prior to generating the generalized model. The quality of the publication and the primary dataset source may be assessed by considering the number of participants analyzed, the risk of bias through standardized procedures, the absence of conflict of interests by the authors, and the like.

In an aspect of the method the characterizing features and the associated factors are adjusted, ranked and/or selected for the conditional probability of an outcome associated with a single characterizing feature across the plurality of publications. In other words, a summary view of the significance of same characterizing feature across the plurality of publications and/or a mixture of publications and probabilistic analyses of primary clinical data is provided.

The plurality of publications subjected to the method described above may be limited to those publications in which the adverse health condition has been mentioned, or considered, irrespective of whether a positive, negative or no correlation between the first adverse health condition and the patient's properties analyzed in the publication has been found. This may serve as a pre-selection for enhancing the quality of the prediction, or for accelerating the execution of the method. Thus, in an aspect of the method, as a further step, the result of a first run of the method, e.g. a characterizing features correlated with an adverse health condition, including those found across the plurality of the publications that was not apparent from any single publication taken alone, may be taken as an input for re-running the method including those publications that had previously not been considered, e.g. in order to find an unlikely and previously unknown correlation between an adverse health condition and a further characterizing feature.

Similarly, in an aspect of the method described above a periodic or event-triggered check for new publications is executed, e.g. by scanning publications for the occurrence of a term or expression or synonym for the adverse health condition or one or more of the characterizing features previously identified, which are related to the adverse health condition. Whenever such new publication is found data is extracted in the way described further above, and the data is input to the method for re-running the steps for generating the generalized model.

The method allows for easy iterative improvement by adding new aggregate data to the initial evidence base extracted from clinical studies, real world evidence, or pieces of literature. Such continuous fine-tuning of the existing model is feasible because the model generation process can be solely based on population summary measures. The prominent advantage of this literature-based analysis approach compared traditional data-driven approaches is that it is optimized to fully rely on published, publicly available aggregated data for model derivation or update and does not need enumerating and following up a new cohort for model derivation or update, an expensive, time-consuming and often unfeasible endeavor. Even though not strictly necessary, analysis of primary clinical data may optionally be used to complement the model obtained through literature review. The model parameters can be easily re-calculated using the same methods as described in the sections above, by simply adding the new aggregate data measure extracted from any eligible new study to the full evidence base previously used to derive the model.

Several aspects of the method described hereinbefore may be combined with each other and/or may be integrated in the underlying method. E.g., the two-run aspect may be combined with adjusting, ranking and/or selecting characterizing features.

Once the generalized model has been generated it may be used in a computer-implemented method of adaptively predicting occurrence or progression of a first adverse health condition for an arbitrarily selectable first subpopulation of a total population. In an aspect this method includes receiving one or more characterizing features from a generalized model generated and stored in a computer accessible and readable memory in accordance with the method of generating a generalized model for adaptively predicting occurrence or progression of a first adverse health condition arbitrarily selected from a total population described further above. The characterizing features may be those identified as risk factors, which have at least some correlation with the adverse health condition, either beneficial or aggravating.

The method of adaptively predicting occurrence or progression of a first adverse health condition for an arbitrarily selectable first subpopulation of a total population further includes receiving data characterizing the first subpopulation. The data characterizing the first subpopulation may comprise all or a subset of the characterizing features required, provided, used, analyzed or considered in the generation of the generalized model. The data characterizing the first subpopulation may thus include data representing physiological features, demographic information, comorbidities, complications and medications, e.g. age, sex, intake of alcohol, smoking, BMI, history of hypertension, diabetes, CKD stage, history of cerebrovascular disease, coronary artery disease, peripheral artery disease, chronic heart failure, chronic obstructive pulmonary disease, autoimmune disorders, anxiety/depression, cancer, liver disease, levels of Albumin, Glucose, HDL, LDL, Triglycerides, CRP, IL-6, S-Uric Acid, HsTNT, Phosphate, iPTH, proteinuria and Albuminuria. It is to be noted that the first subpopulation for this method need not be the same first subpopulation that was referred to in the method for generating the generalized model, and that not all characterizing data need to be used or even available.

The method further includes providing the one or more received characterizing features from the generalized model and the data characterizing the subpopulation to a computing module implementing a probabilistic model. The computing module may be implemented as a computer executing corresponding software, either exclusively or in a dedicated thread that runs in parallel to other tasks on the computer. The probabilistic model may be one of a plurality of known models, e.g. a Bayesian network. The probabilistic model produces, or calculates, as an output signal, a summary score indicating a risk, or a probability, of occurrence or progression of the adverse health condition for the first subpopulation. The risk, or probability, may be provided as an absolute value, or as a relative value, e.g. over a population in which all risk factors are absent.

The output signal is provided to a user or a computer. Providing may include displaying the result on a display screen, printing the result, acoustically providing the result, e.g. over a loudspeaker after text-to-speech conversion, and the like. Providing may also include transmitting the result over a digital communication channel to a user's computer.

In an aspect the method may include providing data signals representing positive or negative effect of one or more characterizing features on the summary score, or the probability of occurrence or progression of the adverse health condition.

In an aspect of the method, in case multiple characterizing features and their effect on the summary score or probability are provided, these are provided in a ranked order according to the significance of their respective contribution to the summary score for the first subpopulation under assessment, i.e. for a single patient or for a group of patients. The ranking may be by absolute or relative contribution. The contribution of a characterizing factor may be either beneficial, i.e. reducing the risk, or adverse, i.e. increasing the risk, and the ranking by significance may be by the absolute significance, irrespective of being beneficial or adverse, or grouped into beneficial and adverse characterizing factors.

In an aspect of the method characterizing features having positive or negative effect on the risk determined for the adverse health condition, which are modifiable through one or more responsive actions from the non-exclusive list comprising therapy, change of lifestyle, change of diet and medical intervention, are highlighted. Highlighting may include printing in bold letters, italics, different font, different font or background color. Selective highlighting may be applied, e.g. in accordance with a ranking of how difficult a modification of a diet or a lifestyle will be for the subpopulation, or how likely an enduring adherence to the modified diet or lifestyle is. Other rankings for the highlighting may include an estimated cost for the modification, e.g. in connection with a selection of possible therapies or medical interventions.

In an aspect the method further includes providing information about how much the risk or the probability of occurrence or progression of the adverse health condition is modified by a responsive action, e.g. by indicating that stopping smoking will reduce the risk by a certain percentage.

In an aspect the method further includes providing a selection of therapy recommendations based on the kind of adverse health condition and/or the risk score. The selection of therapy recommendations may, for example, include closer monitoring of a patient or a subpopulation, e.g., by means of telemedicine, portable devices for monitoring bodily functions, more frequent visits to a physician or a clinic and the like.

In an aspect the method further includes suggesting a selection of additional diagnostic testing and/or therapy that could produce data related to or describing further characterizing features, results or data relating to which can be provided as an input to the method, i.e. the software module, for improving the accuracy of the summary score. The selection of additional testing may be provided in a ranked order, e.g. in accordance with their level of invasiveness to a patient and/or cost, their availability in a region or location, a time until test results can be expected, and the like.

Another aspect of the present disclosure includes a medical device, e.g. implemented by a computer system executing computer program instructions which implement one or more aspects of the methods describe hereinbefore. The medical device may include a display and a user interface, an interface for receiving digital data, and one or more microprocessors and associated volatile and/or non-volatile memory. The interface for receiving digital data may be of conventional type and may provide connection with wired or wireless networks such as Ethernet (LAN), according to standards of the IEEE 802.11 family, also known under the trademark name 'WiFi', according to standards of the IEEE802.15 family, also known under the registered brand name 'Bluetooth', but also with portable data storage devices connected through serial or parallel connections, such as Universal Serial Bus (USB) or a connection according to IEEE 1394 (FireWire). Each of these interfaces comprises a physical transmitter and receiver part as well as a logical transmitter and receiver part, some of which may be similar in structure and operation across the various standards. The medical device may further include a database containing data records associating a plurality of medical risks and a plurality of health parameters and/or characterizing features, as well as other data describing the total population and/or a plurality of subpopulations. The data base may further provide access to a plurality of publications and other medical documentation.

According to one aspect of the present disclosure the medical device, when the processor executes the computer program instructions, may be configured to generate a generalized model for adaptively predicting occurrence or progression of a first adverse health condition for a first subpopulation arbitrarily selected from a total population. The generalized model may represent interrelationships between a plurality of medical risks and a plurality of health parameters. In accordance with this aspect, the medical device is configured to extract information about characterizing features of a plurality of second subpopulations, about occurrences and/or severity of the first adverse health condition and/or about corresponding prognostic results found in a plurality of publications. The publications may, e.g., be obtained through accessing one or more databases. The medical device may further be configured to associate, from each of the plurality of publications, one or more of the characterizing features identified therein with corresponding first factors indicating a relation with an adverse or beneficial contribution of the characterizing feature to the occurrence or progression of the first adverse health condition, and further to associate one or more of the characterizing features with corresponding second factors indicating the relative frequency of occurrence in the respective second subpopulation considered in the respective publication. The medical device may yet further be configured to combine the characterizing features and their first and second factors into a generalized model for the total population, wherein combining includes calculating a baseline risk of patient, and to store the generalized model in a retrievable manner on a computer accessible and readable medium.

In an aspect of the present disclosure the medical device may be configured to perform automatic extraction of information from publications using one or more of electronic text processing, optical character recognition, and natural language processing.

In an aspect of the present disclosure the medical device may be configured to adjust, rank and/or select characterizing features and the associated factors for the quality of the publication prior to generating the generalized model. Adjusting, ranking and/or selecting may be executed in accordance with the quality of the publication, or in accordance with the conditional probability of an outcome associated with a single characterizing feature across the plurality of publications.

In an aspect of the present disclosure the medical device may be configured to, in a first run of the method, limit the plurality of publications to those considering the first adverse health condition and, in a second run of the method, to use the result of the first run as an input to the method, along with one or more publications not considering the first adverse health condition.

According to one aspect of the of the present disclosure the medical device, when the processor executes the computer program instructions, may be configured to adaptively predict occurrence or progression of a first adverse health condition for an arbitrarily selectable first subpopulation of a total population. In accordance with this aspect, the medical device is configured to receive one or more characterizing features from a generalized model generated and stored in a computer accessible and readable memory, e.g. in accordance with the method described further above. The medical device is further configured to receive data characterizing the first subpopulation and to provide the one or more received characterizing features from the generalized model and the data characterizing the subpopulation to a software module implementing a probabilistic model. The medical device is yet further configured to provide a summary score output from the software module, indicating a risk or probability of occurrence or progression of the adverse health condition for the first subpopulation, and to provide, to a user, one or more characterizing features and their positive or negative effect on the risk or the probability of occurrence or progression of the adverse health condition.

In an aspect of the present disclosure the medical device may be configured to provide the one or more characterizing features and their positive or negative effect on the risk or the probability of occurrence or progression of the adverse health condition in a ranked order according to their significance of their contribution to the summary score.

In an aspect of the present disclosure the medical device may be configured to highlight those characterizing features having positive or negative effect on the risk or the probability of occurrence or progression of the adverse health condition which can be modified or influenced through one or more responsive actions from the non-exclusive list comprising therapy, change of lifestyle, change of diet and medical intervention.

In an aspect of the present disclosure the medical device may be configured to provide information about how much the risk or the probability of occurrence or progression of the adverse health condition is modified by a responsive action.

In an aspect of the present disclosure the medical device may be configured to provide a selection of therapy recommendations based on the kind of adverse health condition and/or the risk score.

In an aspect of the present disclosure the medical device may be configured to provide a selection of additional diagnostic tests and/or therapy that could produce data related to or describing further characterizing features, results or data relating to which can be provided to the software module for improving the accuracy of the summary score. The selection of additional diagnostic tests and/or therapy may be provided in a ranked order, e.g. in accordance with their cost, availability within a region or location, a time until test results are available or visible effects can be expected, and the like.

Another aspect of the present disclosure includes a computer-readable medium for use on a computer system configured to perform generating a generalized model for adaptively predicting occurrence or progression of a first adverse health condition for a first subpopulation arbitrarily selected from a total population. The computer-readable medium according to this aspect may have computer-executable instructions for performing a method including extracting information about characterizing features of a plurality of second subpopulations, about occurrences and/or severity of the first adverse health condition found therein and/or about corresponding prognostic results, from a plurality of publications, associating one or more of the characterizing features identified in each of the plurality of publications with corresponding first factors indicating a relation with an adverse or beneficial contribution of the characterizing feature to the occurrence or progression of the first adverse health condition, further associating one or more of the characterizing features with corresponding second factors indicating the relative frequency of occurrence in the respective second subpopulation considered in the respective publication, combining the characterizing features and their first and second factors into a generalized model for the total population, wherein combining includes calculating a baseline risk of patient, and storing the generalized model in a retrievable manner on a computer accessible and readable medium.

Yet another aspect of the present disclosure includes a computer-readable medium for use on a computer system configured to perform adaptively predicting occurrence or progression of a first adverse health condition for an arbitrarily selectable first subpopulation of a total population. The computer-readable medium according to this aspect may have computer-executable instructions for performing a method including receiving one or more characterizing features from a generalized model generated and stored in a computer accessible and readable memory, receiving data characterizing the first subpopulation, providing the one or more received characterizing features from the generalized model and the data characterizing the subpopulation to a software module implementing a probabilistic model, providing a summary score from the software module, indicating a risk or probability of occurrence or progression of the adverse health condition for the first subpopulation and providing, to a user, one or more characterizing features and their positive or negative effect on the risk or the probability of occurrence or progression of the adverse health condition.

Storing, receiving and/or providing as used throughout this specification may include establishing a physical and/or logical digital communication channel between a processor and a memory, between a first and a second computer over a digital communication connection or network, or combinations thereof.

The present medical devices and methods may provide efficient and accurate prediction of adverse health conditions for an arbitrarily selected subpopulation or individual based on health information obtained from publications, literature and the like that focus on a limited or selected subpopulation. Such technology may be used to predict and manage individual health risks as well as to analyze and manage health risks of a group or a population.

Patient-specific or subpopulation-specific ranking of risk factors and beneficial factors by their impact on the absolute or relative risk allows for a physician or other medical caregiver to provide more focused and more effective treatments or preventive measures.

Individual users may use the disclosed medical devices and methods to predict occurrence or progression of potential adverse health conditions based on their own health data or characterizing features. The individual users may also obtain information for reducing the risks or the likelihood of occurrence or progression of an adverse health condition changing relevant behavior, e.g., lifestyle, corresponding to its contribution to the risk or likelihood.

Group or institutional users may use the disclosed medical devices and methods to calculate health risks among a population, such as a particular distribution among the population. The institutional users may also optimize the distribution to reduce the health risks of a population and to promote healthy lifestyle.

**Brief description of the drawings**

In the following section the method will be described with reference to the drawings. In the drawings

Fig. 1     illustrates a flowchart diagram of an exemplary method 100 of generating a generalized model for adaptively predicting occurrence or progression of a first adverse health condition for a first subpopulation arbitrarily selected from a total population,

Fig. 2     describes a more detailed, exemplary embodiment of the selection step shown in figure 1,

Fig. 3     illustrates a more detailed, exemplary embodiment of the merging and computation steps shown in figure 1,

Fig. 4     depicts a data and results flow diagram of an exemplary method of predicting occurrence or progression of a first adverse health condition for an arbitrarily selectable first subpopulation of a total population,

Fig. 5     shows a flowchart diagram of one exemplary aspect of the method of generating a generalized model for adaptively predicting occurrence or progression of a first adverse health condition for a first subpopulation arbitrarily selected from a total population, in which the generalized model is updated by analyzing new publications, and

Fig. 6     shows an exemplary computer system adapted to perform one or more aspects of the present method.

**Description of Embodiments**

Reference will now be made in detail to exemplary embodiments, which are illustrated in the accompanying drawings. Wherever possible, the same reference numbers will be used throughout the drawings to refer to the same or like parts.

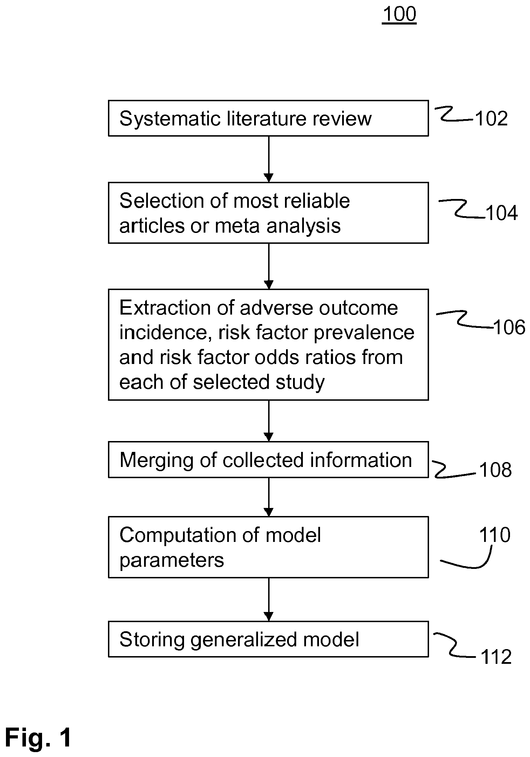

Figure 1 illustrates a flowchart diagram of an exemplary method of generating a generalized model for adaptively predicting occurrence or progression of a first adverse health condition for a first subpopulation arbitrarily selected from a total population. In step 102 literature or other publications, including medical records from hospitals or other health institutions, are systematically reviewed, and in step 104 the most reliable articles or meta analyses from the literature or publications are selected. From these selected documents characterizing features, e.g., adverse outcome incidence, risk factor prevalence and risk factor odds ratios, are extracted in step 106 and merged in step 108. The merged information is then used in step 110 to compute model parameters for the generalized model, which are stored in step 112. The model parameters may be computed using any appropriate type of probabilistic model, e.g., exponential family functions.

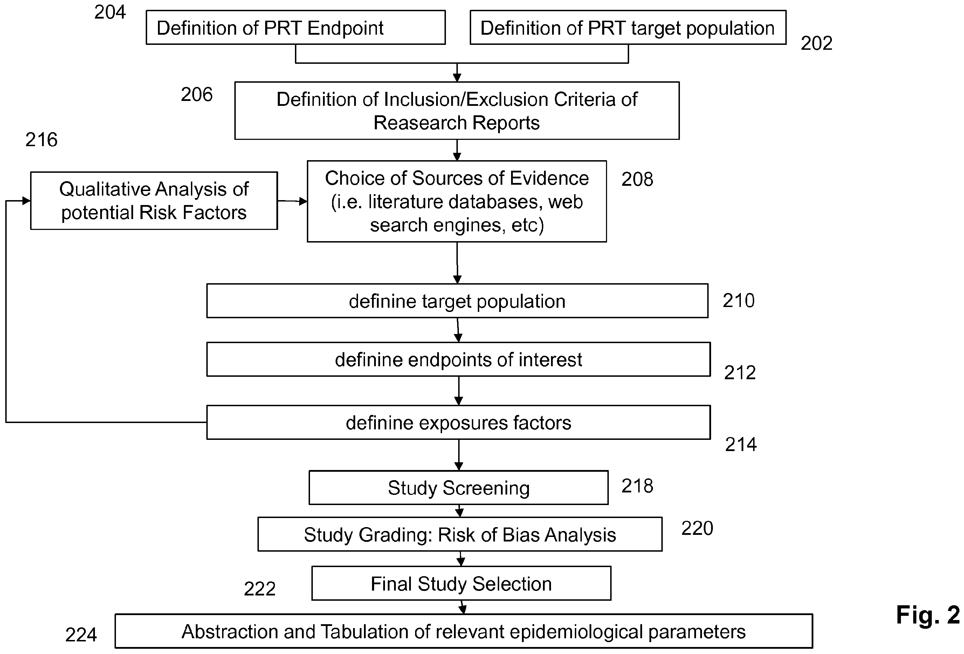

Figure 2 describes a more detailed, exemplary embodiment of steps 102-106 shown in figure 1. In steps 202 and 204 the target population and the endpoint of the prognostic reasoning tool are defined and forwarded as input to step 206, in which inclusion and exclusion criteria for the publications are defined. Steps 208 to 216, entail defining the sources of evidence, and search terms defining the target population, endpoints of interest, and characterizing features. At the end of each cycle the literature is qualitatively assessed in order to identify potential new exposure/risk factors.

Once the search strategy and all required definitions are made the publications are screened and graded in steps 218 and 220, respectively, and a final selection of publications for analysis and extraction is made in step 222. The extraction of characterizing features etc. is then performed in step 224.

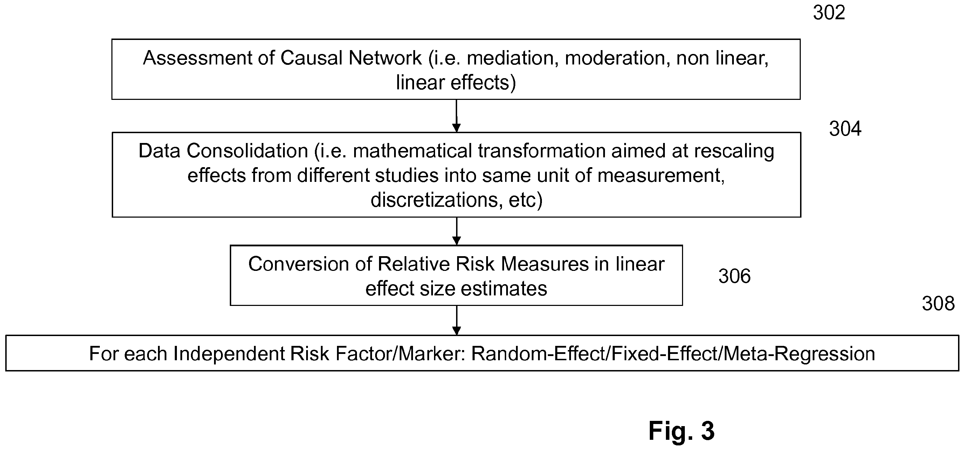

Figure 3 illustrates a more detailed, exemplary embodiment of merging and computation steps 108 and 110, respectively, shown in figure 1. In step 302 an assessment is performed to generate a theory representing the process of causation linking the exposure and the outcome of interest. In steps 304 and 306 the measures of association extracted from the literature are consolidated into a common effect size measure. This may include scaling for different sample sizes, different discretization and the like. At this point, different meta-analytic techniques can be performed for each independent risk factor or marker in step 308.

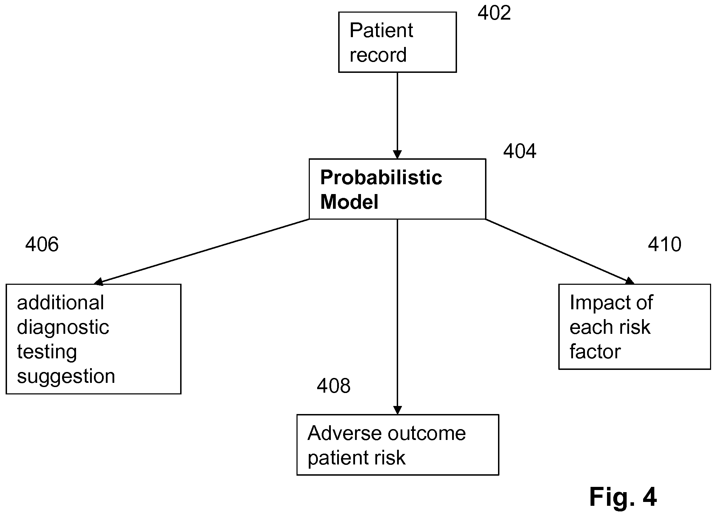

Figure 4 depicts a data and results flow diagram of an exemplary method of predicting occurrence or progression of a first adverse health condition for an arbitrarily selectable first subpopulation of a total population. In the example the subpopulation is a single patient. Data 402 from a patient record is provided to the generalized probabilistic model 404. Running the analysis using the generalized probabilistic model 404 provides one or more of a suggestion 406 for additional diagnostic testing, an indication representing a patient risk for an adverse health condition 408 and an indication 410 how big the impact of each characterizing feature, or risk factor, is on the patient risk for acquiring or progress of an adverse health condition.

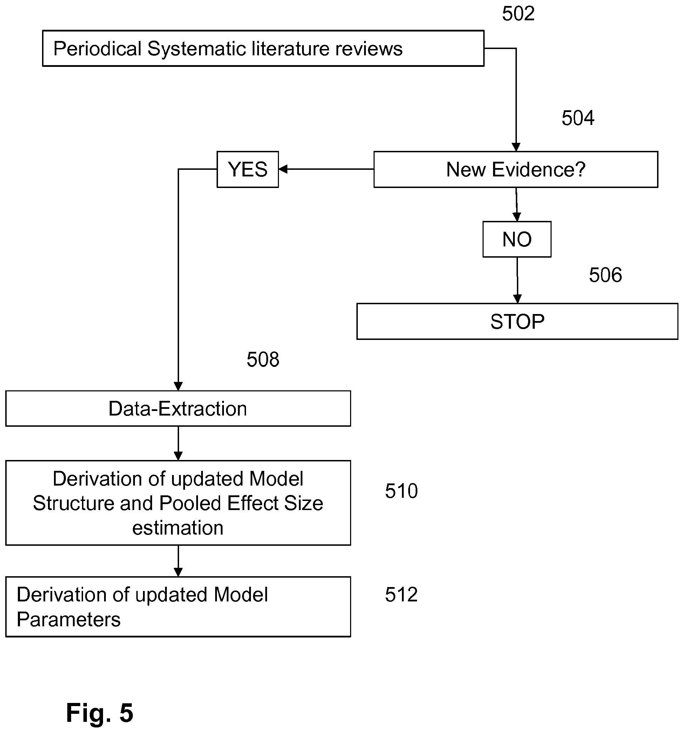

Figure 5 shows a flowchart diagram of one exemplary aspect of the method of generating a generalized model for adaptively predicting occurrence or progression of a first adverse health condition for a first subpopulation arbitrarily selected from a total population, in which the generalized model is updated by analyzing new publications. Periodical or event-triggered check for new publications is executed in step 502. The review may be performed in the same way as in step 102 of figure 1, and appropriate keywords may be determined in the same way as explained with reference to figure 2. Whenever a publication is found in step 504 that promises to provide new evidence or generally new data in respect of an adverse health condition, e.g. new subpopulations, new characteristic features, etc., the method follows the "yes"-path to step 508, in which the data extraction is executed, e.g. in the way discussed with reference to figure 1. The following steps 510 and 512 likewise may correspond to one or more of steps 106 to 110 discussed with reference to figure 1. Updating the generalized model using new publications allows for the model to become more accurate or effective in predicting without having to derive again the full model generation process that was initially required and thus saves time and effort.

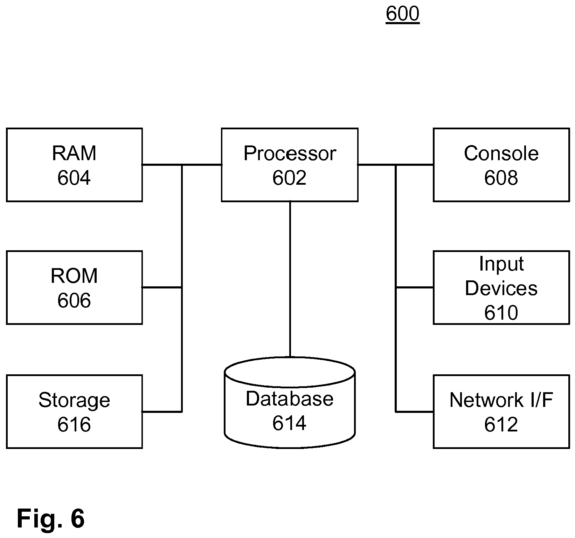

Figure 6 shows an exemplary computer system 600 adapted to perform one or more aspects of the present method. Computer system 600 may include a processor 602, a random access memory (RAM) 604, a read-only memory (ROM) 606, a console 608, input devices 610, network interfaces 612, database 614, and a storage 616. It is understood that the type and number of listed devices are exemplary only and not intended to be limiting. The number of listed devices may be changed and other devices may be added.

Processor 602 may include any appropriate type of general purpose microprocessor, digital signal processor, or microcontroller. Processor 602 may execute sequences of computer program instructions to perform various processes or method steps as explained above. The computer program instructions may be loaded into RAM 604 for execution by processor 602 from a read-only memory (ROM), or from storage 616. Storage 616 may include any appropriate type of mass storage provided to store any type of information that processor 602 may need to perform the processes. For example, storage 616 may include one or more hard disk devices, optical disk devices, or other storage devices to provide storage space.

Console 608 may provide a graphic user interface (GUI) to display information to users of computer system 600. Console 608 may include any appropriate type of computer display device or computer monitor. Input devices 610 may be provided for users to input information into computer system 600. Input devices 610 may include a keyboard, a mouse, or other optical or wireless computer input devices, etc. Further, network interfaces 612 may provide communication connections such that computer system 600 may be accessed remotely through computer networks via various communication protocols, such as transmission control protocol/internet protocol (TCP/IP), hyper text transfer protocol (HTTP), etc.

Database 614 may contain model data and/or any information related to data records under analysis, such as model parameters and testing data. Databases 614 may include any type of commercial or customized databases. Databases 614 may also include analysis tools for analyzing the information in the database. Processor 602 may also use databases 614 to determine and store performance characteristics of the generalized model.

Other embodiments, features, aspects, and principles of the disclosed exemplary medical devices and methods will be apparent to those skilled in the art and may be implemented in various environments and systems.

## Claims

1. A computer-implemented method of generating a generalized model for adaptively predicting occurrence or progression of a first adverse health condition for a first subpopulation arbitrarily selected from a total population, including:

   - extracting information about characterizing features of a plurality of second subpopulations, about occurrences and/or severity of the first adverse health condition found therein and/or about corresponding prognostic results, from a plurality of publications and/or primary clinical data sources;
   - from each of the plurality of publications and/or primary clinical data sources, associating one or more of the characterizing features identified therein with corresponding first factors indicating a relation with an adverse or beneficial contribution of the characterizing feature to the occurrence or progression of the first adverse health condition, and further associating one or more of the characterizing features with corresponding second factors indicating the relative frequency of occurrence in the respective second subpopulation considered in the respective publication;

- combining the characterizing features and their first and second factors into a generalized model for the total population, wherein combining includes calculating a baseline risk of patient; and
- storing the generalized model in a retrievable manner on a computer accessible and readable medium.

2. The method of claim 1, wherein extracting includes controlling an automatic extraction module implementing one or more of electronic text processing, optical character recognition, and natural language processing.

3. The method of claim 1 or 2, further including adjusting, ranking and/or selecting the characterizing features and the associated factors in accordance with a value representing a quality of the publication and/or primary clinical data source prior to generating the generalized model.

4. The method of any one of claims 1 to 3, further including adjusting, ranking and/or selecting the characterizing features and the associated factors in accordance with a value representing the conditional probability of an outcome associated with a single characterizing feature across the plurality of publications and/or primary clinical data sources.

5. The method of any one of claims 1 to 4, further including, in a first run of the method, limiting the plurality of publications and/or primary clinical data sources to those considering the first adverse health condition and, in a second run of the method, using the result of the first run as an input to the method, along with one or more publications and/or primary clinical data sources not considering the first adverse health condition.

6. A computer-implemented method of adaptively predicting occurrence or progression of a first adverse health condition for an arbitrarily selectable first subpopulation of a total population, including:

- receiving one or more characterizing features from a generalized model generated and stored in a computer accessible and readable memory in accordance with the method of one or more of claims 1 to 7;
- receiving data characterizing the first subpopulation;
- providing the one or more received characterizing features from the generalized model and the data characterizing the subpopulation to a software module implementing a probabilistic model;
- providing a summary score from the software module, indicating a risk or probability of occurrence or progression of the adverse health condition for the first subpopulation; and
- providing, to a user, one or more characterizing features and their positive or negative effect on the risk or the probability of occurrence or progression of the adverse health condition.

7. The method of claim 6, further including providing the one or more characterizing features and their positive or negative effect on the risk or the probability of occurrence or progression of the adverse health condition in a ranked order according to their significance of their contribution to the summary score.

8. The method of claim 7, further including highlighting those characterizing features having positive or negative effect on the risk or the probability of occurrence or progression of the adverse health condition which can be modified or influenced through one or more responsive actions from the non-exclusive list comprising therapy, change of lifestyle, change of diet and medical intervention.

9. The method of claim 8, further including providing information about how much the risk or the probability of occurrence or progression of the adverse health condition is modified by a responsive action.

10. The method of any one of claims 6 to 9, further including providing a selection of therapy recommendations based on the kind of adverse health condition and/or the risk score.

11. The method of any one of claims 6 to 10, further including providing a selection of additional diagnostic tests and/or therapy that could produce data related to or describing further characterizing features, results or data relating to which can be provided to the software module for improving the accuracy of the summary score.

12. The method of claim 10 or 11, further including providing the selection of additional diagnostic tests and/or therapy in a ranked order.

13. A system for generating a generalized model for adaptively predicting occurrence or progression of a first adverse health condition for a first subpopulation arbitrarily selected from a total population, including:

- an extraction module configured to receive first data signals representing a plurality of publications and/or primary clinical data sources, to extract, from the data signals, information about characterizing features of a plurality of second subpopulations, about occurrences and/or severity of the first adverse health condition found therein and/or about corresponding prognostic results, from the plurality of publications and/or primary clinical data sources, and to generate corresponding second data signals;

- an association module configured to receive the second data signals, to associate one or more of the characterizing features identified in each of the plurality of publications and/or primary clinical data sources and represented by the second data signals with corresponding first factors indicating a relation with an adverse or beneficial contribution of the characterizing feature to the occurrence or progression of the first adverse health condition, to generate corresponding third data signals, further to associate one or more of the characterizing features with corresponding second factors indicating the relative frequency of occurrence in the respective second subpopulation considered in the respective publication and/or primary clinical data source, and to generate corresponding fourth data signals;

- a combination module configured to receive the second, third and fourth data signals, to combine the characterizing features and their first and second factors, represented by the second, third and fourth data signals, into a generalized model for the total population, wherein combining includes calculating a baseline risk of patient, and to provide a fifth data signal representing the generalized model; and

- a data access module configured to access a computer accessible and readable medium and to store the fifth data signal representing the generalized model therein in a retrievable manner.

14. A system for adaptively predicting occurrence or progression of a first adverse health condition for an arbitrarily selectable first subpopulation of a total population, including:

- a first data receiver module configured to receive data signals representing one or more characterizing features from a generalized model generated and stored in a computer accessible and readable memory in accordance with the method of one or more of claims 1 to 7;

- a second data receiver module configured to receive data signals representing a characterization of the first subpopulation;

- a software module configured to receive and process the data signals representing the one or more received characterizing features from the generalized model and the data signals representing a characterization of the subpopulation, the software module including an implementation of a probabilistic model, and configured to provide a summary score from the software module, indicating a risk or probability of occurrence or progression of the adverse health condition for the first subpopulation; and

- a providing module configured to provide, to a user or a further medical device, one or more characterizing features and their positive or negative effect on the risk or the probability of occurrence or progression of the adverse health condition.

<u>100</u>

```
┌─────────────────────────────────┐
│ Systematic literature review    │  ⟜—102
└─────────────────────────────────┘
              │
              ▼
┌─────────────────────────────────┐
│ Selection of most reliable      │  ⟜—104
│ articles or meta analysis       │
└─────────────────────────────────┘
              │
              ▼
┌─────────────────────────────────┐
│ Extraction of adverse outcome   │
│ incidence, risk factor prevalence│ ⟜—106
│ and risk factor odds ratios from│
│ each of selected study          │
└─────────────────────────────────┘
              │
              ▼
┌─────────────────────────────────┐
│ Merging of collected information│  ⟜—108
└─────────────────────────────────┘
              │
              ▼
┌─────────────────────────────────┐
│ Computation of model            │  110
│ parameters                      │
└─────────────────────────────────┘
              │
              ▼
┌─────────────────────────────────┐
│ Storing generalized model       │  ⟜—112
└─────────────────────────────────┘
```

# Fig. 1

204 — Definition of PRT Endpoint

Definition of PRT target population — 202

206 — Definition of Inclusion/Exclusion Criteria of Reasearch Reports

216 — Qualitative Analysis of potential Risk Factors

Choice of Sources of Evidence (i.e. literature databases, web search engines, etc) — 208

definine target population — 210

definine endpoints of interest — 212

definine exposures factors — 214

Study Screening — 218

Study Grading: Risk of Bias Analysis — 220

222 — Final Study Selection

224 — Abstraction and Tabulation of relevant epidemiological parameters

**Fig. 2**

EP 3 471 107 A1

302

Assessment of Causal Network (i.e. mediation, moderation, non linear, linear effects)

304

Data Consolidation (i.e. mathematical transformation aimed at rescaling effects from different studies into same unit of measurement, discretizations, etc)

Conversion of Relative Risk Measures in linear effect size estimates          306

308

For each Independent Risk Factor/Marker: Random-Effect/Fixed-Effect/Meta-Regression

**Fig. 3**

EP 3 471 107 A1

Patient
record

402

Probabilistic
Model

404

406

additional
diagnostic
testing
suggestion

410

Impact of
each risk
factor

408

Adverse outcome
patient risk

**Fig. 4**

502

```
Periodical Systematic literature reviews
```

504

```
New Evidence?
```

YES ←

NO

506

```
STOP
```

508

```
Data-Extraction
```

```
Derivation of updated Model
Structure and Pooled Effect Size
estimation
```
510

```
Derivation of updated Model
Parameters
```
512

## Fig. 5

600

| | | |
|---|---|---|
| RAM 604 | Processor 602 | Console 608 |
| ROM 606 | | Input Devices 610 |
| Storage 616 | Database 614 | Network I/F 612 |

**Fig. 6**

Europäisches
Patentamt

European
Patent Office

Office européen
des brevets

## EUROPEAN SEARCH REPORT

Application Number

EP 17 19 6137

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 563 654 A1 (HOECHST ROUSSEL PHARMA [US]) 6 October 1993 (1993-10-06) * paragraph [0037] - paragraph [0040] * * paragraph [0066] * * paragraph [0094] * * paragraph [0113] * * paragraph [0123] * * figures 2-3 * | 1-14 | INV. G16H50/50 G16H50/80 |
| X | US 2014/095201 A1 (FAROOQ FAISAL [US] ET AL) 3 April 2014 (2014-04-03) * page 4, line 17 - line 25 * | 1-14 | |
| A | Suhail A. R. Doi and Gail M. Williams: "Methods of Clinical Epidemiology", 14 May 2014 (2014-05-14), Springer, XP002779161, ISBN: 9783642371318 * page 147 - page 161 * | 1 | |
| A | M. CHOY ET AL: "Estimating disease prevalence using census data", EPIDEMIOLOGY AND INFECTION., vol. 136, no. 09, 30 November 2007 (2007-11-30), XP055460540, GB ISSN: 0950-2688, DOI: 10.1017/S0950268807009752 * page 1254, column 1, paragraph 2 - page 1255, column 1, paragraph 2 * | 1 | TECHNICAL FIELDS SEARCHED (IPC) G16H |

-/--

The present search report has been drawn up for all claims

| Place of search | Date of completion of the search | Examiner |
|---|---|---|
| Munich | 27 March 2018 | Rivera, Pedro V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 17 19 6137

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CARLO DAVILA-PAYAN ET AL: "Estimating Prevalence of Overweight or Obese Children and Adolescents in Small Geographic Areas Using Publicly Available Data", PREVENTING CHRONIC DISEASE, vol. 12, 1 January 2015 (2015-01-01), page 140229, XP055461131, DOI: 10.5888/pcd12.140229 * the whole document * | 1 | |

-----

TECHNICAL FIELDS
SEARCHED       (IPC)

The present search report has been drawn up for all claims

| Place of search | Date of completion of the search | Examiner |
|---|---|---|
| Munich | 27 March 2018 | Rivera, Pedro V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 17 19 6137

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2018

| Patent document cited in search report | | Publication date | Patent family member(s) | | Publication date |
|---|---|---|---|---|---|
| EP 0563654 | A1 | 06-10-1993 | CA<br>EP | 2091828 A1<br>0563654 A1 | 19-09-1993<br>06-10-1993 |
| US 2014095201 | A1 | 03-04-2014 | NONE | | |

EPO FORM P0459

For more details about this annex : see Official Journal of the European Patent Office, No. 12/82